# EUROPEAN PATENT APPLICATION

(11) **EP 4 166 565 A1**
(43) Date of publication of application: **19.04.2023**
(21) Application number: 21828935.3
(22) Date of filing: 24.06.2021
(51) Int. Cl.: C07K 14/32, C12N 5/10, C12N 1/15, C12N 1/19, C12N 1/21, C12N 15/31, C12N 15/52, C12N 15/63, C12N 9/00, C12P 21/02

(54) **METHOD FOR PRODUCING DIPEPTIDE**

(30) Priority: 25.06.2020 JP 2020109616
(71) Applicant: Kyowa Hakko Bio Co., Ltd., Tokyo 100-8185 (JP)
(72) Inventor: TABATA, Kenichiro, Tokyo 100-8185 (JP); TSUDA, Naoto, Tokyo 100-8185 (JP); ADACHI,Yuugo, Tokyo 100-8185 (JP)
(74) Representative: Vossius & Partner Patentanwälte Rechtsanwälte mbB
(86) International application number: PCT/JP2021/023994
(87) International publication number: WO 2021/261564

(57) **Abstract**

An object of the present invention is to provide a protein having dipeptide synthesizing activity with improved substrate specificity, and a method in which the protein or a microorganism having ability to produce the protein is used to efficiently produce a target dipeptide while reducing a by-product dipeptide produced in addition to the target dipeptide. According to the present invention, a protein consisting of an amino acid sequence obtained by substituting, with other amino acid residues, amino acid residues corresponding to one or more amino acid residues selected from the group consisting of amino acid residues at positions 107, 108, and 110 in an amino acid sequence set forth in SEQ ID NO: 2, or a mutant protein or a homologous protein of a protein consisting of the amino acid sequence set forth in SEQ ID NO: 2 is provided, and a microorganism producing the protein can be used to efficiently produce a dipeptide.

## Description

### Technical Field

The present invention relates to a protein having dipeptide synthesizing activity with improved substrate specificity, and a method in which the protein or a microorganism having ability to produce the protein is used to efficiently produce a target dipeptide while reducing a by-product dipeptide produced in addition to the target dipeptide.

### Background Art

It has been reported that a microorganism belonging to the genus *Bacillus* has L-amino acid α ligase that produces, from ATP and two molecules of L-amino acids, a dipeptide having a peptide-bond at a carboxyl group in the α position (Patent Literature 1 and Non Patent Literature 1). L-amino acid α ligase can synthesize a dipeptide without needing modification of amino acid and a special coenzyme, and hence is very useful for efficient production of a dipeptide. L-amino acid α ligase has, however, low substrate specificity, and hence has a problem that dipeptides are produced as by-products in addition to a target peptide. For example, Non Patent Literature 2 discloses a method in which a DNA encoding a protein YwfE derived from *Bacillus subtilis* and having L-amino acid α ligase activity is transformed into *E.coli*, and the resultant transformant is used for producing L-alanyl-L-glutamine, and it has been reported that L-alanyl-L-alanine is significantly produced at this point as a by-product dipeptide in addition to L-alanyl-L-glutamine.

As a characteristic of YwfE, it is known that L-asparagine at position 108, L-glutamic acid at position 109, and L-leucine at position 110 in the amino acid sequence of YwfE are positioned in the vicinity of C-terminal amino acids of a dipeptide that is a product (Non Patent Literature 3). Among these, L-glutamic acid at position 109 makes contribution to interaction with a magnesium ion significant for enzyme activity of YwfE (Non Patent Literature 3). A residue corresponding to the L-glutamic acid is widely stored in L-amino acid α ligase contained in various microorganisms (Non Patent Literature 4), and therefore, it is suggested that the L-glutamic acid can be a significant amino acid residue in enzyme activity of L-amino acid α ligase.

Considerable studies have been made on YwfE modified in substrate specificity (Patent Literatures 2 to 6, and Non Patent Literature 3).

Patent Literature 2 describes that a dipeptide having an acidic amino acid on the N terminal side can be efficiently produced by modifying, L-phenylalanine at position 62, L-isoleucine at position 92, L-tryptophan at position 332, L-asparagine at position 333, L-methionine at position 334, L-aspartic acid at position 359, and L-aspartic acid at position 361 in the amino acid sequence of YwfE.

Patent Literature 3 describes that carnosine can be efficiently produced by modifying, L-isoleucine at position 112 and L-histidine at position 378 in the amino acid sequence of YwfE.

Patent Literature 4 describes that a dipeptide having a basic amino acid on the N terminal side can be efficiently produced by modifying, L-asparagine at position 107, L-aspartic acid at position 283, L-tryptophan at position 332, and L-aspartic acid at position 376 in the amino acid sequence of YwfE.

Patent Literature 5 describes that a dipeptide having a branched chain amino acid on the N terminal side can be efficiently produced by modifying L-methionine at position 334 in the amino acid sequence of YwfE.

Patent Literature 6 describes that imidazole peptide can be efficiently produced by modifying, L-asparagine at position 108, L-isoleucine at position 112, and L-histidine at position 378 in the amino acid sequence of YwfE.

Non Patent Literature 3 describes that synthesizing activity of prolyl glycine of Tab S is improved by substituting, in L-amino acid ligase Tab S contained in *Pseudomonas syringae,* serine at position 85 corresponding to an amino acid residue at position 110 in the amino acid sequence of YwfE with threonine.

On the other hand, it has not been reported that the modification of a specific amino acid residue in the amino acid sequence of YwfE improves the substrate specificity of L-amino acid α ligase to reduce the production of a by-product dipeptide produced in addition to a target dipeptide.

### Citation List

### Patent Literature

Patent Literature 1: International Publication No. WO2004/058960
Patent Literature 2: Japanese Unexamined Patent Publication No. 2013-81404
Patent Literature 3: Japanese Unexamined Patent Publication No. 2013-81405
Patent Literature 4: Japanese Unexamined Patent Publication No. 2013-81406
Patent Literature 5: Japanese Unexamined Patent Publication No. 2013-81407
Patent Literature 6: Japanese Unexamined Patent Publication No. 2018-102287

### Non Patent Literature

Non Patent Literature 1: Kazuhiko Tabata et al., "ywfE in Bacillus subtilis Codes for a Novel Enzyme, L-Amino Acid Ligase", J. Bacteriol., 187, p. 5195-5202 (2005)
Non Patent Literature 2: Kazuhiko Tabata et al., "Fermentative Production of L-Alanyl-L-Glutamine by a Metabolically Engineered Escherichia coli Strain Expressing L-Amino Acid α-Ligase", Appl. Environ. Microbiol., 73, 20, p. 6378-6385 (2007)
Non Patent Literature 3: Yasuhito Shomura, "Structural and enzymatic characterization of BacD, an L-amino acid dipeptide ligase from Bacillus subtilis", Protein Science, 21, p. 707-716 (2012)
Non Patent Literature 4: Haruka Kino, "Effective production of Pro-Gly by mutagenesis of l-amino acid ligase", J. Biosci. Bioen., 122, 2, p. 155-159 (2016)

### Summary of Invention

### Technical Problem

An object of the present invention is to provide a protein having dipeptide synthetase enzyme activity, and a dipeptide production method in which the protein or a microorganism having ability to produce the protein is used to improve the substrate specificity of the protein to thereby efficiently produce a target dipeptide while reducing a by-product dipeptide produced in addition to the target dipeptide.

### Solution to Problem

The present invention relates to the following 1 to 8:
1. A protein consisting of an amino acid sequence obtained by substituting one or more amino acid residues selected from the group consisting of amino acid residues at positions 107, 108, and 110 in an amino acid sequence set forth in SEQ ID NO: 2 with amino acid residues of the following [1] to [3], respectively, the protein having L-amino acid α ligase activity with improved substrate specificity as compared with an original protein having the amino acid sequence set forth in SEQ ID NO: 2:
   [1] for the amino acid residue at position 107, an amino acid residue selected from the group consisting of L-serine, L-alanine, L-histidine, L-glutamine, L-aspartic acid, L-glutamic acid, glycine, and L-methionine;
   [2] for the amino acid residue at position 108, an amino acid residue selected from the group consisting of L-phenylalanine, L-methionine, L-serine, L-proline, L-threonine, L-alanine, L-tyrosine, glycine, and L-leucine; and
   [3] for the amino acid residue at position 110, an amino acid residue selected from the group consisting of L-methionine, L-valine, L-alanine, L-asparagine, L-cysteine, L-tryptophan, and L-phenylalanine.
2. A protein consisting of an amino acid sequence obtained by substituting one or more amino acid residues selected from the group consisting of amino acid residues corresponding to positions 107, 108, and 110 in an amino acid sequence of an original protein according to the following [4] or [5] with amino acid residues of the following [1'] to [3'], respectively, the protein having L-amino acid α ligase activity with improved substrate specificity as compared with the original protein:
   [1'] for the amino acid residue at position 107, an amino acid residue selected from the group consisting of L-serine, L-alanine, L-histidine, L-glutamine, L-aspartic acid, L-glutamic acid, glycine, and L-methionine;
   [2'] for the amino acid residue at position 108, an amino acid residue selected from the group consisting of L-phenylalanine, L-methionine, L-serine, L-proline, L-threonine, L-alanine, L-tyrosine, glycine, and L-leucine; and
   [3'] for the amino acid residue at position 110, an amino acid residue selected from the group consisting of L-methionine, L-valine, L-alanine, L-asparagine, L-cysteine, L-tryptophan, and L-phenylalanine;
   [4] a mutant protein consisting of an amino acid sequence obtained by deleting, substituting, inserting, and/or adding 1 to 20 amino acids in an amino acid sequence set forth in SEQ ID NO: 2, and having L-amino acid α ligase activity; and
   [5] a homologous protein consisting of an amino acid sequence having 80% or more identity to the amino acid sequence set forth in SEQ ID NO: 2, and having L-amino acid α ligase activity.
3. A DNA encoding the protein according to the above 1 or 2.
4. A recombinant DNA comprising the DNA according to the above 3.
5. A transformant obtained by transforming a host cell with the recombinant DNA according to the above 4 described.
6. A method for producing a dipeptide, comprising, with the protein according to the above 1 or 2 used as an enzyme source, causing the enzyme source and two types of L-amino acids to be present in an aqueous medium, thereby producing and accumulating a dipeptide in the aqueous medium, and collecting the dipeptide from the aqueous medium.
7. A method for producing a dipeptide, comprising culturing, in a medium, a microorganism having ability to produce the protein according to the above 1 or 2, thereby producing and accumulating a dipeptide in a culture, and collecting the dipeptide from the culture.
8. The production method according to the above 6 or 7, wherein the dipeptide is represented by the formula (I):

   R¹-R² (I)

   wherein R¹ represents L-alanine, and R² represents an amino acid residue selected from L-glutamine, L-glutamic acid, glycine, L-valine, L-leucine, L-isoleucine, L-proline, L-phenylalanine, L-tryptophan, L-methionine, L-serine, L-threonine, L-cysteine, L-asparagine, L-tyrosine, L-lysine, L-arginine, L-histidine, and L-aspartic acid.

### Advantageous Effects of Invention

According to the present invention, a protein having dipeptide synthesizing activity with improved substrate specificity, and a production method in which a microorganism having ability to produce the protein is used to efficiently produce a specific dipeptide while reducing a by-produced dipeptide can be provided.

### Description of Embodiments

### 1. Protein of the present invention

A protein of the present invention is a protein according to the following item (1) or (2):
(1) A protein consisting of an amino acid sequence obtained by substituting one or more amino acid residues selected from the group consisting of amino acid residues at positions 107, 108, and 110 in an amino acid sequence set forth in SEQ ID NO: 2 with amino acid residues of the following [1] to [3], respectively, and having L-amino acid α ligase activity with improved substrate specificity as compared with an original protein having the amino acid sequence set forth in SEQ ID NO: 2:
   [1] for the amino acid residue at position 107, an amino acid residue selected from the group consisting of L-serine, L-alanine, L-histidine, L-glutamine, L-aspartic acid, L-glutamic acid, glycine, and L-methionine;
   [2] for the amino acid residue at position 108, an amino acid residue selected from the group consisting of L-phenylalanine, L-methionine, L-serine, L-proline, L-threonine, L-alanine, L-tyrosine, glycine, and L-leucine; and
   [3] for the amino acid residue at position 110, an amino acid residue selected from the group consisting of L-methionine, L-valine, L-alanine, L-asparagine, L-cysteine, L-tryptophan, and L-phenylalanine.
(2) A protein consisting of an amino acid sequence obtained by substituting, in an amino acid sequence of an original protein according to the following [4] or [5], one or more amino acid residues selected from the group consisting of amino acid residues corresponding to positions 107, 108, and 110 in an amino acid sequence set forth in SEQ ID NO: 2 with amino acid residues of the following [1'] to [3'], respectively, and having L-amino acid α ligase activity with improved substrate specificity as compared with the original protein according to [4] or [5]:
   [1'] for the amino acid residue corresponding to position 107, an amino acid residue selected from the group consisting of L-serine, L-alanine, L-histidine, L-glutamine, L-aspartic acid, L-glutamic acid, glycine, and L-methionine;
   [2'] for the amino acid residue corresponding to position 108, an amino acid residue selected from the group consisting of L-phenylalanine, L-methionine, L-serine, L-proline, L-threonine, L-alanine, L-tyrosine, glycine, and L-leucine; and
   [3'] for the amino acid residue corresponding to position 110, an amino acid residue selected from the group consisting of L-methionine, L-valine, L-alanine, L-asparagine, L-cysteine, L-tryptophan, and L-phenylalanine;
   [4] a mutant protein consisting of an amino acid sequence obtained by deleting, substituting, inserting, and/or adding 1 to 20 amino acids in the amino acid sequence set forth in SEQ ID NO: 2, and having L-amino acid α ligase activity; and
   [5] a homologous protein consisting of an amino acid sequence having 80% or more identity to the amino acid sequence set forth in SEQ ID NO: 2, and having L-amino acid α ligase activity.

The original protein described above in item (2) is the mutant protein described in item [4] or the homologous protein described in item [5]. Amino acid residues in the amino acid sequence of the original protein corresponding to the positions 107, 108, and 110 in the amino acid sequence set forth in SEQ ID NO: 2 refer to respective amino acid residues aligned in the same positions as the amino acid residues at positions 107, 108, and 110 in the amino acid sequence of SEQ ID NO: 2 when the amino acid sequence of the original protein and the amino acid sequence of SEQ ID NO: 2 are aligned against each other. Hereinafter, the amino acid residues at positions 107, 108, and 110 in the amino acid sequence set forth in SEQ ID NO: 2, and the amino acid residues corresponding to the positions 107, 108, and 110 in the amino acid sequence set forth in SEQ ID NO: 2 are both referred to as the "amino acid residues corresponding to the positions 107, 108, and 110 in the amino acid sequence set forth in SEQ ID NO: 2".

In the amino acid sequence of the original protein described above in item (2), the amino acid residues corresponding to the positions 107, 108, and 110 in the amino acid sequence set forth in SEQ ID NO: 2 refer to amino acid residues at positions 107, 108, and 110 in an amino acid sequence set forth in SEQ ID NO: 3 when the original protein is a protein consisting of the amino acid sequence set forth in SEQ ID NO: 3 having 80% or more identity to the amino acid sequence set forth in SEQ ID NO: 2.

In the amino acid sequence of the protein described above in item (1) or (2), it can be confirmed that one or more amino acid residues selected from the group consisting of the amino acid residues corresponding to the positions 107, 108, and 110 in the amino acid sequence set forth in SEQ ID NO: 2 are substituted with the amino acid residues described above in item [1] to [3] of (1) or [1'] to [3'] of (2) by aligning the amino acid sequence of the protein described above in item (1) or (2) with the amino acid sequence of the original protein.

Alignment of an amino acid sequence can be created, for example, by using a known alignment program ClustalW [Nucleic Acids Research 22, 4673 (1994)]. ClustalW is available from, for example, http://www.ebi.ac.uk/clustalw/ (European Bioinformatics Institute). As a parameter to be used in creating alignment by using ClustalW, for example, a default value can be used.

The amino acid residue corresponding to the position 107 in the amino acid sequence set forth in SEQ ID NO: 2 is substituted with preferably an amino acid residue selected from the group consisting of L-serin, L-alanine, L-histidine, glycine, and L-methionine, and more preferably an amino acid residue selected from the group consisting of L-alanine, L-histidine, and glycine among the amino acids described above in item [1] or [1'].

The amino acid residue corresponding to the position 108 in the amino acid sequence set forth in SEQ ID NO: 2 is substituted with preferably an amino acid residue selected from the group consisting of L-phenylalanine, L-methionine, L-serin L-threonine, L-alanine, L-tyrosine, and L-leucine, and more preferably an amino acid residue selected from the group consisting of L-serine, L-threonine, L-alanine, and L-tyrosine among the amino acids described above in item [2] or [2'].

The amino acid residue corresponding to the position 110 in the amino acid sequence set forth in SEQ ID NO: 2 is substituted with preferably an amino acid residue selected from the group consisting of L-methionine, L-cysteine, L-tryptophan, and L-phenylalanine, and more preferably an amino acid residue selected from the group consisting of L-methionine, L-tryptophan, and L-phenylalanine among the amino acids described above in item [3] or [3'].

L-amino acid α ligase activity refers to activity for producing, by using ATP and different types of two molecules of L-amino acids as a substrate, a dipeptide in which the two types of amino acids are peptide-bonded to each other at a carboxyl group in the α position. Here, L-amino acids include glycine having no stereoisomers.

Examples of the L-amino acid to be used as a substrate of the protein having L-amino acid α ligase activity include L-alanine, L-asparagine, L-aspartic acid, L-glutamine, L-glutamic acid, glycine, L-histidine, L-isoleucine, L-leucine, L-lysine, L-arginine, L-methionine, L-phenylalanine, L-proline, L-serine, L-threonine, L-tryptophan, L-tyrosine, L-valine, and L-cysteine.

It can be confirmed, for example, by the following method, that the protein described above in item (1) or (2) has improved substrate specificity as compared with the original protein. First, a recombinant DNA having a DNA encoding the protein to be confirmed for the activity is produced by a method described below. Next, the recombinant DNA is used for culturing a microorganism having no L-amino acid α ligase activity, for example, a microorganism obtained by transforming *Escherichia coli* W3110 strain, and a cell extract containing the protein is prepared from the thus obtained culture. The cell extract containing the protein is contacted with an aqueous solution containing the substrate, that is, ATP and two types of L-amino acids, and thus, dipeptides are produced in the aqueous solution. Ultimately, the dipeptides contained in the reaction solution are detected by HPLC described below, and amounts of a target dipeptide and by-product dipeptides produced are compared with each other, and thus, it can be confirmed that the substrate specificity is improved as compared with that in the original protein.

A mutant protein refers to a protein obtained by artificially deleting or substituting an amino acid residue in an original protein, or artificially inserting or adding an amino acid residue in the protein.

In the mutant protein described in item [4], for deleting, substituting, inserting or adding the amino acids, 1 to 20 amino acids may be deleted, substituted, inserted, or added in optional positions in the amino acid sequence set forth in SEQ ID NO: 2, and for example, 1 to 15, 1 to 10, or 1 to 5 amino acids may be deleted, substituted, inserted, or added.

The amino acids to be substituted, inserted, or added may be naturally occurring or non-naturally occurring amino acids. Examples of the naturally occurring amino acids include L-alanine, L-asparagine, L-aspartic acid, L-glutamine, L-glutamic acid, glycine, L-histidine, L-isoleucine, L-leucine, L-lysine, L-arginine, L-methionine, L-phenylalanine, L-proline, L-serine, L-threonine, L-tryptophan, L-tyrosine, L-valine, and L-cysteine.

Examples of mutually substitutable amino acids are as follows. Amino acids belonging to the same group are mutually substitutable.
Group A: leucine, isoleucine, norleucine, valine, norvaline, alanine, 2-aminobutanoic acid, methionine, o-methylserine, t-butylglycine, t-butylalanine, and cyclohexylalanine
Group B: aspartic acid, glutamic acid, isoaspartic acid, isoglutamic acid, 2-aminoadipic acid, and 2-aminosuberic acid
Group C: asparagine, and glutamine
Group D: lysine, arginine, ornithine, 2,4-diaminobutanoic acid, and 2,3-diaminopropionic acid
Group E: proline, 3-hydroxyproline, and 4-hydroxyproline
Group F: serine, threonine, and homoserine
Group G: phenylalanine, and tyrosine

Homologous proteins refer to a group of proteins that every living thing in nature has, and are derived from proteins having the same evolutionary origin. The homologous proteins are similar to one another in the structure and the function.

The amino acid sequence of the homologous protein described in item [5] has, to the amino acid sequence set forth in SEQ ID NO: 2, at least 80% or more, preferably 85% or more, more preferably 90% or more, further preferably 95% or more, and most preferably 98% or more identity.

Identity between amino acid sequences or nucleotide sequences can be determined by using algorithm BLAST by Karlin and Altschul [Pro. Nat. Acad. Sci. USA, 90, 5873 (1993)] or FASTA [Methods Enzymol., 183, 63 (1990)]. Based on the algorithm BLAST, programs designated as BLASTN or BLASTX have been developed [J. Mol. Biol., 215, 403 (1990)]. When a nucleotide sequence is to be analyzed by using BLASTN based on BLAST, parameters are set, for example, as score = 100, and wordlength = 12. Alternatively, when an amino acid sequence is to be analyzed by using BLASTX based on BLAST, parameters are set, for example, as score = 50, and wordlength = 3. When BLAST and Gapped BLAST program are used, default parameters of the respective programs are used. Specific methods for performing these analysis methods are known.

Specific examples of the homologous protein includes protein of *Bacillus amyloliquefaciens* set forth in SEQ ID NO: 3 (Uniprot KB-Q8KWS8).

It can be confirmed, for example, by the following method, that the mutant protein or the homologous protein has L-amino acid α ligase activity. First, a recombinant DNA having a DNA encoding the mutant protein or the homologous protein to be confirmed for the activity is produced by a method described below. Next, the recombinant DNA is used for culturing a microorganism having no L-amino acid α ligase activity, for example, a microorganism obtained by transforming *Escherichia coli* W3110, and a cell extract containing the protein is prepared from the thus obtained culture. The cell extract containing the protein is contacted with an aqueous solution containing the substrate, that is, ATP and two types of L-amino acids, and thus, dipeptides are produced in the aqueous solution. Ultimately, the dipeptides contained in the reaction solution are detected by HPLC described below, and thus, it can be confirmed that the mutant protein or the homologous protein has L-amino acid α ligase activity.

### 2. DNA of the present invention

A DNA of the present invention is a DNA encoding the protein described above in item (1) or (2).

An example of the DNA of the present invention specifically includes a DNA encoding a protein consisting of an amino acid sequence obtained by substituting, in an amino acid sequence of a protein encoded by a DNA described in any one of the following items [6] to [8], one or more amino acid residues selected from the group consisting of the amino acid residues corresponding to the positions 107, 108, and 110 in the amino acid sequence set forth in SEQ ID NO: 2 with the amino acid residues described above in item [1] to [3] or [1'] to [3']:
[6] a DNA consisting of a nucleotide sequence set forth in SEQ ID NO: 1;
[7] a DNA that hybridizes a DNA consisting of a nucleotide sequence complementary to the nucleotide sequence set forth in SEQ ID NO: 1 under stringent conditions, and encodes a mutant protein or a homologous protein having L-amino acid α ligase activity; and
[8] a DNA consisting of a nucleotide sequence having at least 80% or more, preferably 90% or more, further preferably 95% or more, and most preferably 98% or more identity to the nucleotide sequence set forth in SEQ ID NO: 1, and encodes a mutant protein or a homologous protein having L-amino acid α ligase activity.

In the above description, to "hybridize" refers to a step of hybridizing the DNA with a DNA having a specific nucleotide sequence or a part of the DNA. Accordingly, a nucleotide sequence of the DNA that hybridizes the DNA having a specific nucleotide sequence or a part of the DNA may be a DNA that is useful as a probe for Northern or Southern blot analysis, or has a length usable as an oligonucleotide primer in PCR analysis.

An example of a DNA used as a probe includes a DNA having at least 100 bases or more, preferably 200 bases or more, and more preferably 500 bases or more in length, and an example of a DNA used as a primer includes a DNA having at least 10 bases or more, and preferably 15 bases or more in length.

A method of DNA hybridization experiment is well known, and the experiment can be performed with hybridization conditions determined in accordance with, for example, Molecular Cloning, 4th edition (Cold Spring Harbor Laboratory Press (2012)), Methods for General and Molecular Bacteriology (ASM Press (1994)), Immunology methods manual (Academic Press (1997)), and other many standard textbooks.

Alternatively, also in accordance with a manual attached to a commercially available hybridization kit, a DNA that hybridizes under stringent conditions can be obtained. An example of the commercially available hybridization kit includes Random Primed DNA Labeling Kit (manufactured by Roche Diagnostic) with which a probe is prepared by a random prime method to perform hybridization under stringent conditions.

The stringent conditions can be, for example, the following conditions: A filter having a DNA immobilized thereon and a probe DNA are incubated at 42°C overnight in a solution containing 50% formamide, 5 x SSC (750 mM sodium chloride and 75 mM sodium citrate), 50 mM sodium phosphate (pH 7.6), 5 x Denhardt's solution, 10% dextran sulfate, and 20 µg/L modified salmon sperm DNA, and the resultant filter is washed in, for example, 0.2 x SSC solution at about 65°C.

The above-described various conditions can be set also by adding or changing a blocking reagent used for suppressing the background of the hybridization experiment. The addition of the blocking reagent may include change of the hybridization conditions for conforming the conditions.

An example of the DNA hybridizable under the stringent conditions includes a DNA consisting of a nucleotide sequence having at least 80% or more, preferably 90% or more, further preferably 95% or more, and most preferably 98% or more identity, which is calculated using the above-described program such as BLAST or FASTA based on the above-descried parameters, to the nucleotide sequence set forth in SEQ ID NO: 1.

The DNA of the present invention can be obtained, for example, by mutating, with a DNA encoding the protein having the amino acid sequence set forth in SEQ ID NO: 2, a nucleotide sequence of a portion, on the DNA, encoding one or more amino acid residues selected from the group consisting of amino acid residues corresponding to the positions 107, 108, and 110 in the amino acid sequence set forth in SEQ ID NO: 2 by a site directed mutagenesis method described in, for example, Molecular Cloning, 4th edition (Cold Spring Harbor Laboratory Press (2012)), Current Protocols in Molecular Biology (JOHN WILEY & SONS, INC.) or the like to substitute the nucleotide sequence with a nucleotide sequence encoding another amino acid residue. Alternatively, the DNA of the present invention can be obtained by using Prime STAR Mutagenesis Basal Kit (manufactured by Takara Bio Inc.) or the like.

The DNA of the present invention can be obtained in a similar manner by mutating, for example, with a DNA encoding a mutant protein consisting of an amino acid sequence obtained by deleting, substituting, inserting and/or adding 1 to 20 amino acids in the amino acid sequence set forth in SEQ ID NO: 2, and having L-amino acid α ligase activity, a nucleotide sequence of a portion encoding one or more amino acid residues selected from the group consisting of the amino acid residues corresponding to the positions 107, 108, and 110 in SEQ ID NO: 2 in an amino acid sequence of the mutant protein in alignment of the amino acid sequence of the mutant protein with the original amino acid sequence set forth in SEQ ID NO: 2 by the method described above in item 1.

Alternatively, the DNA of the present invention can be obtained by mutating, with a DNA consisting of an amino acid sequence having 80% or more identity to the amino acid sequence set forth in SEQ ID NO: 2, and encoding a homologous protein having L-amino acid α ligase activity, a nucleotide sequence of a portion encoding one or more amino acid residues selected from the amino acid residues corresponding to the positions 107, 108, and 110 of SEQ ID NO: 2 in an amino acid sequence of the homologous protein in alignment of the amino acid sequence of the homologous protein against the original amino acid sequence set forth in SEQ ID NO: 2 by the method described above in item 1.

A DNA encoding a protein having the amino acid sequence set forth in SEQ ID NO: 2 can be obtained, for example, by PCR [PCR Protocols, Academic Press (1990)] using, as a template, a chromosomal DNA of *Bacillus subtilis* 168 (ATCC 23857) strain in which a probe designable based on a nucleotide sequence of a DNA encoding a protein having the amino acid sequence set forth in SEQ ID NO: 2 is used; Southern hybridization in chromosomal DNA library of a microorganism, preferably one belonging to the genus *Bacillus*, and more preferably *Bacillus subtilis* 168 (ATCC 23857) is employed; or a primer DNA designable based on a DNA encoding the protein having the amino acid sequence set forth in SEQ ID NO: 2 is used. A specific example of the DNA encoding the protein having the amino acid sequence set forth in SEQ ID NO: 2 includes a DNA having the nucleotide sequence set forth in SEQ ID NO: 1.

The DNA encoding the mutant protein, described above in item [4] of (2) in 1, consisting of an amino acid sequence obtained by deleting, substituting, inserting, and/or adding 1 to 20 amino acids in the amino acid sequence set forth in SEQ ID NO: 2, and having L-amino acid α ligase activity can be obtained, for example, by performing error-prone PCR or the like using the DNA consisting of the nucleotide sequence set forth in SEQ ID NO: 1 as a template.

Alternatively, the DNA encoding the mutant protein, described above in item [4] of (2) in 1, consisting of an amino acid sequence obtained by deleting, substituting, inserting, and/or adding 1 to 20 amino acids in the amino acid sequence set forth in SEQ ID NO: 2, and having L-amino acid α ligase activity can be obtained by site directed mutagenesis [Gene, 77, 51 (1989)] by PCR using a pair of PCR primers respectively having, at the 5' terminals, a nucleotide sequence designed to introduce target mutation (deletion, substitution, insertion, or addition).

Alternatively, the DNA can be obtained in accordance with a manual attached to a commercially available site directed mutagenesis kit. An example of the commercially available site directed mutagenesis kit includes Prime STAR(R) Mutagenesis Basal Kit (manufactured by Takara Bio Inc.) capable of introducing mutation (deletion, substitution, insertion, or addition) in a position into which target mutation is desired to introduce.

Specifically, first, a pair of mutation introducing primers having 15 bases overlapped on the 5' side is designed by using, as a template, a plasmid having a nucleotide sequence designed to introduce target mutation (deletion, substitution, insertion, or addition). At this point, the overlap portion includes the target mutation. Next, the mutation introducing primers are used to perform PCR by using, as a template, a plasmid having a nucleotide sequence to which the target mutation is desired to be introduced. When the thus obtained amplified fragment is transformed into *E. coli,* a plasmid having a nucleotide sequence in which the target mutation has been introduced can be obtained.

A DNA encoding the homologous protein, described above in item [5] of (2) in 1, consisting of an amino acid sequence having 80% or more identity to the amino acid sequence set forth in SEQ ID NO: 2, and having L-amino acid α ligase activity can be obtained, for example, by the following method. Specifically, a DNA encoding the homologous protein can be obtained by a method similar to a method in which a nucleotide sequence having 80% or more, preferably 90% or more, more preferably 95% or more, and most preferably 99% or more identity to the nucleotide sequence set forth in SEQ ID NO: 1 is searched for in various gene sequence database, and a probe DNA or a primer DNA designable based on a nucleotide sequence or an amino acid sequence obtained through the search, and a microorganism having the DNA are used to obtain the DNA encoding the protein having the amino acid sequence set forth in SEQ ID NO: 2.

The identity between nucleotide sequences or amino acid sequences can be determined in the same manner as described above in item 1.

The DNA of the present invention obtained by any of the above-described methods is incorporated, directly or after cleaving with an appropriately restriction enzyme, into a vector by a conventional method, the thus obtained recombinant DNA is introduced into a host cell, and then, analysis is performed by a usually employed nucleotide sequence analysis method such as deoxy method [Proc. Nat. Aca. Sci., USA, 74, 5463 (1977)], or with a nucleotide sequence analyzer such as Applied Biosystems 3500 Genetic Analyzer or Applied Biosystems 3730 DNA analyzer (both manufactured by Thermo Fisher Scientific), and thus, the nucleotide sequence of the DNA can be determined.

Examples of the vector usable in determining the nucleotide sequence of the DNA of the present invention include pBluescript II KS (+), and pPCR-Script Amp SK (+) (both manufactured by Agilent Technologies Japan, Ltd.), pT7 Blue (manufactured by Merck Millipore), pCRII (manufactured by Thermo Fisher Scientific), pCR-TRAP (manufactured by Gene Hunter), and pDIRECT [Nucleic Acids Res., 18, 6069 (1990)].

The host cell may be any cell as long as it can be amplified with the vector introduced thereinto, and examples include *Escherichia coli* DH5a, *Escherichia coli* HST08 Premium, *Escherichia coli* HST02, *Escherichia coli* HST04 dam-/dcm-, *Escherichia coli* JM109, *Escherichia coli* HB101, *Escherichia coli* CJ236, *Escherichia coli* BMH71-18 mutS, *Escherichia coli* MV1184, and *Escherichia coli* TH2 (all manufactured by Takara Bio Inc.), *Escherichia coli* XL1-Blue, and *Escherichia coli* XL2-Blue (both manufactured by Agilent Technologies Japan, Ltd.), *Escherichia coli* DH1, *Escherichia coli* MC1000, *Escherichia coli* W1485, *Escherichia coli* W3110, *Escherichia coli* MP347, and *Escherichia coli* NM522.

As a method for introducing the recombinant DNA, obtained by incorporation of the DNA of the present invention, into the host cell, any methods for introducing a DNA into a host cell can be employed, and examples include a method using a calcium ion [Proc. Natl. Acad. Sci., USA, 69, 2110 (1972)], Protoplast method (Japanese Unexamined Patent Publication No. S63-248394), and electroporation method [Nucleic Acids Res., 16, 6127 (1988)].

As a result of the determination of the nucleotide sequence, when the obtained DNA is a partial length DNA, a full length DNA can be obtained by Southern Hybridization in chromosomal DNA library using the partial length DNA as a probe, or the like.

Besides, based on the determined nucleotide sequence of the DNA, a target DNA can be prepared by chemical synthesis using NTSM series DNA Synthesizer manufactured by Nihon Techno Service Co., Ltd., or the like

### 3. Recombinant DNA of the present invention

A recombinant DNA of the present invention is a DNA autonomous replicable in a host cell, and is a DNA in which the DNA of the present invention is incorporated into an expression vector containing a promoter in position where the DNA of the present invention described above in item 2 can be transcribed.

The recombinant DNA of the present invention is a DNA that can be incorporated into a chromosome in a host cell, and a DNA having the DNA of the present invention is also the recombinant DNA of the present invention.

When the recombinant DNA is a recombinant DNA that can be incorporated into a chromosome, a promoter may not be contained therein.

When a prokaryote such as a bacterium is used as the host cell, the recombinant DNA of the present invention is preferably a recombinant DNA containing a promoter, a ribosome binding sequence, the DNA of the present invention described above in item 2, and a transcription termination sequence. A gene for controlling the promoter may be further contained therein.

Here, a distance between a Shine-Dalgarno sequence, that is, a ribosome binding sequence, and a start codon is preferably adjusted to an appropriate distance, such as 6 to 18 bases.

Besides, in the recombinant DNA of the present invention, a transcription termination sequence is not always necessary for expressing the DNA of the present invention, but a transcription termination sequence is preferably placed directly below a structural gene.

When a microorganism belonging to the genus *Escherichia* is used as the host cell into which the recombinant DNA of the present invention is to be introduced, the expression vector can be, for example, pColdI, pSTV28, and pUC118 (all manufactured by Takara Bio Inc.), pET21a, pCDF-1b, and pRSF-1b (all manufactured by Merck Millipore), pMAL-c5x (manufactured by New England Biolabs, Inc.), pGEX-4T-1, and pTrc99A (both manufactured by GE Healthcare Bioscience), pTrcHis, and pSE280 (both manufactured by Thermo Fisher Scientific), pGEMEX-1 (manufactured by Promega Corp.), pQE-30, pQE-60, and pQE80L (all manufactured by Qiagen K.K.), pET-3, pBluescriptII SK(+), and pBluescriptII KS(-) (all manufactured by Agilent Technologies Japan, Ltd.), pKYP10 (Japanese Unexamined Patent Publication No. S58-110600), pKYP200 [Agric. Biol. Chem., 48, 669 (1984)], pLSA1[Agric. Biol. Chem., 53, 277 (1989)], pGEL1 [Proc. Natl. Acad. Sci., USA, 82, 4306 (1985)], pTrS30 [prepared from *Escherichia coli* JM109/pTrS30 (FERM BP-5407)], pTrS32 [prepared from *Escherichia coli* JM109/pTrS32 (FERM BP-5408)], pTK31 [APPLIED AND ENVIRONMENTAL MICROBIOLOGY, 2007, Vol. 73, No. 20, p. 6378-6385], pPE167 (Appl. Environ. Microbiol. 2007, 73: 6378-6385), pPAC31 (WO98/12343), pUC19 [Gene, 33, 103 (1985)], pPA1 (Japanese Unexamined Patent Publication No. S63-233798), and the like.

When any of the above-described expression vectors is used, the promoter may be any promoter as long as it functions in a cell of a microorganism belonging to the genus *Escherichia,* and, for example, a promoter derived from *Escherichia coli,* a phage or the like, such as a trp promoter, a gapA promoter, a lac promoter, a PL promoter, a PR promoter, or a PSE promoter, can be used. Besides, an artificially designed/modified promoter, such as a promoter containing two tandem trp promoters, a tac promoter, a trc promoter, a lac T5 promoter, a lac T7 promoter, or a letI promoter, can be used.

When a coryneform bacterium is used as the host cell into which the recombinant DNA of the present invention is to be introduced, the expression vector can be, for example, pCG1 (Japanese Unexamined Patent Publication No. S57-134500), pCG2 (Japanese Unexamined Patent Publication No. S58-35197), pCG4 (Japanese Unexamined Patent Publication No. S57-183799), pCG11 (Japanese Unexamined Patent Publication No. S57-134500), pCG116, pCE54, and pCB101 (all Japanese Unexamined Patent Publication No. S58-105999), pCE51, pCE52, and pCE53 [all Molecular and General Genetics, 196, 175 (1984)], and the like.

When any of the above-described expression vectors is used, the promoter may be any promoter as long as it functions in a cell of a coryneform bacterium, and for example, P54-6 promoter [Appl. Microbiol. Biotechnol., 53, p. 674-679 (2000)] can be used.

When a yeast strain is used as the host cell into which the recombinant DNA of the present invention is to be introduced, the expression vector can be, for example, YEp13 (ATCC37115), YEp24 (ATCC37051), YCp50 (ATCC37419), pHS19, pHS15, and the like.

When any of the above-described expression vectors is used, the promoter may be any promoter as long as it functions in a cell of a yeast strain, and examples include promoters such as a PHOS promoter, a PGK promoter, a GAP promoter, an ADH promoter, a gal1 promoter, a gal10 promoter, a heat shock polypeptide promoter, a MFα1 promoter, and a CUP1 promoter.

The recombinant DNA of the present invention can be produced, for example, by treating a DNA fragment prepared by the method described above in item 2 with a restriction enzyme to insert the resultant into downstream of the promoter of any of the above-described appropriate expression vectors.

Here, when bases are substituted in the nucleotide sequence of the DNA of the present invention to obtain an optimal codon for expression of the host cell, the expression level of the protein encoded by the DNA can be also improved. Information on codon usage in the host cell is available through public database.

### 4. Transformant of the present invention

A transformant of the present invention is a transformant obtained by transforming a host cell with the recombinant DNA of the present invention described above in item 3, namely, the recombinant DNA containing the DNA of the present invention described above in item 2.

The host cell into which the recombinant DNA of the present invention is to be introduced may be any one of a prokaryote, a yeast, an animal cell, an insect cell, a plant cell, and the like, examples include preferably a prokaryote and a yeast strain, more preferably prokaryotes belonging to the genus *Escherichia,* the genus *Serratia*, the genus *Bacillus*, the genus *Brevibacterium*, the genus *Corynebacterium,* the genus *Microbacterium,* and the genus *Pseudomonas,* and yeast strains belonging to the genus *Saccharomyces,* the genus *Schizosaccharomyces,* the genus *Kluyveromyces,* the genus *Trichosporon,* the genus *Schwanniomyces,* the genus *Pichia,* and the genus *Candida,* and more preferably prokaryotes such as *Escherichia coli* BL21 codon plus, *Escherichia coli* XL1-Blue, and *Escherichia coli* XL2-Blue (all manufactured by Agilent Technologies Japan, Ltd.), *Escherichia coli* BL21(DE3) pLysS (manufactured by Merck Millipore), *Escherichia coli* DH5a, *Escherichia coli* HST08 Premium, *Escherichia coli* HST02, *Escherichia coli* HST04 dam-/dcm-, *Escherichia coli* JM109, *Escherichia coli* HB101, *Escherichia coli* CJ236, *Escherichia coli* BMH71-18 mutS, *Escherichia coli* MV1184, and *Escherichia coli* TH2 (all manufactured by Takara Bio Inc.), *Escherichia coli* W, *Escherichia coli* JM101, *Escherichia coli* W3110, *Escherichia coli* MG1655, *Escherichia coli* DH1, *Escherichia coli* MC1000, *Escherichia coli* W1485, *Escherichia coli* MP347, *Escherichia coli* NM522, *Escherichia coli* ATCC9637, *Serratia ficaria, Serratia fonticola*, *Serratia liquefaciens*, *Serratia marcescens, Bacillus subtilis, Bacillus amyloliquefaciens*, *Brevibacterium immariophilum* ATCC14068, *Brevibacterium saccharolyticum* ATCC14066, *Corynebacterium ammoniagenes, Corynebacterium glutamicum* ATCC13032, *Corynebacterium glutamicum* ATCC14067, *Corynebacterium glutamicum* ATCC13869, *Corynebacterium acetoacidophilum* ATCC13870, *Microbacterium ammoniaphilum* ATCC15354, and *Pseudomonas* sp. D-0110, and yeast strains such as *Saccharomyces cerevisiae, Schizosaccharomyces pombe, Kluyveromyces lactis, Trichosporon pullulans, Schwanniomyces alluvius, Pichia pastoris,* and *Candida utilis.*

As the host cell, a bred strain artificially reduced in dipeptide degrading activity, a bred strain artificially imparted with or increased in ability to produce L-amino acid to be used as a substrate of a target dipeptide, or a bred strain having been subjected to both of these artificial treatments can be preferably used.

A method for artificially weakening dipeptide degrading activity of a microorganism used as the host cell can be a method in which at least one of enzymes having dipeptide degrading activity is weakened or blocked.

Examples of a method for artificially imparting with or increasing in the ability to produce L-amino acid in a microorganism used as the host cell include a method (a) in which at least one of mechanisms for controlling a biosynthetic pathway for producing target L-amino acid is released or cancelled, a method (b) in which at least one of enzymes involved in the biosynthetic pathway for producing the target L-amino acid is enhanced in expression, a method (c) in which a copy number of at least one of enzyme genes involved in the biosynthetic pathway for producing the target L-amino acid is increased, and a method (d) in which at least one of metabolic pathways branching from the biosynthetic pathway for producing the target L-amino acid to metabolites other than the target substance is weakened or blocked, and these known methods can be singly used, or can be used in combination.

Specific examples of a method for weakening dipeptide degrading activity, and imparting with or increasing in the ability to produce L-amino acid to be used as a substrate include known methods such as methods by various genetic engineering techniques (Appl Environ Microbiol (2007) 73 (20): 6378-6385).

Examples of a method for introducing the recombinant DNA described above in item 3 into a host cell as an autonomous replicable plasmid include the method using a calcium ion, the Protoplast method, and the electroporation method described above, and a spheroplast method [Proc. Natl. Acad. Sci., USA, 81, 4889 (1984)], and a lithium acetate method [J. Bacteriol., 153, 163 (1983)].

An example of a method for incorporating the recombinant DNA into a chromosome of a host cell includes a homologous recombination method. An example of the homologous recombination method includes a method using a homologous recombination plasmid producible to be linked to a plasmid DNA having a drug resistance gene that cannot be autonomously replicated in a host cell into which it is desired to be introduced. An example of a method utilizing homologous recombination, which is frequently used in *Escherichia coli,* includes a method for introducing a recombinant DNA utilizing a homologous recombination system of random phage [Proc. Natl. Acad. Sci. USA, 97, 6641-6645 (2000)].

Besides, *E. coli* in which a target region on a chromosomal DNA of a host cell is substituted with the recombinant DNA can be obtained by a selection method utilizing that *E. coli* attains sucrose sensitivity with *Bacillus subtilis* levansucrase incorporated into a chromosome together with a recombinant DNA, a selection method utilizing that *E. coli* attains streptomycin sensitivity through incorporation of wild type rpsL gene into *E. coli* having streptomycin-resistant mutant rpsL gene [Mol. Microbiol., 55, 137 (2005), Biosci. Biotechnol. Biochem., 71, 2905 (2007)], or the like.

It can be confirmed, by culturing the transformant in a medium to compare an amount of a target dipeptide produced, accumulated in the culture with that of a parent strain, that the transformant obtained by any of the above-described methods has the DNA of the present invention described above in item 2. Alternatively, it can be confirmed by preparing an extract containing the protein of the present invention from the culture, causing the extract and two different types of L-amino acids to be present in an aqueous medium, and comparing an amount of the dipeptide produced, accumulated in the aqueous medium with that of a parent strain.

Examples of such transformant of the present invention include transformants described below in examples.

### 5. Method for Producing Dipeptide of the present invention

A method for producing a dipeptide of the present invention is a method described in the following item 5-1 or 5-2.

### 5-1. Method for Producing Dipeptide using L-amino Acid as Substrate

An example of the method for producing a dipeptide of the present invention includes a method for producing a dipeptide using two types of L-amino acids as precursors.

Specifically, the protein described in item (1) or (2) is used as an enzyme source, the enzyme source and two types of L-amino acids are caused to be present in an aqueous medium, a dipeptide is caused to be produced and accumulated in the aqueous medium, and the dipeptide can be collected from the aqueous medium. The enzyme source may be the protein described in item (1) or (2) having been purified, or the transformant described in item 4, namely, a culture obtained by culturing, in a medium, a microorganism having ability to produce the protein described above in item (1) or (2) having been purified, or a treated product of the culture. When the purified protein described above in item (1) or (2) is used as the enzyme source, ATP is caused to be present in the aqueous solvent together with the two types of L-amino acids. Here, the culture encompasses the cultured microorganism having the ability to produce the protein described above in item (1) or (2) and the medium, and contains the protein described above in item (1) or (2) having been expressed in the culture by the microorganism.

ATP and the two types of L-amino acids may be derived from anything as long as they can be a substrate of the protein of the present invention contained in the transformant of the present invention, and a culture of a microorganism having ability to produce ATP and the two types of L-amino acids, or the treated product of the culture may be directly used, or the ATP and the two types of L-amino acids collected from the culture or the treated product of the culture may be used.

The culture or the treated product of the culture contains the protein described above in item (1) or (2) as the enzyme source, and ATP. Examples of the treated product of the culture include a concentrate of the culture, a dried product of the culture, a bacterial cell obtained by centrifuging the culture, a dried product of the bacterial cell, a freeze dried product of the bacterial cell, a surfactant-treated product of the bacterial cell, a sonicated product of the bacterial cell, a mechanically ground product of the bacterial cell, a solvent-treated product of the bacterial cell, an enzyme-treated product of the bacterial cell, a protein fraction of the bacterial cell, and an enzyme preparation obtained through extraction from an immobilized product of the bacterial cell or the bacterial cell.

Examples of the aqueous medium include buffers such as water, phosphate, carbonate, acetate, borate, citrate, and Tris, alcohols such as methanol and ethanol, esters such as ethyl acetate, ketones such as acetone, and amides such as acetamide. Alternatively, a culture fluid of the microorganism used as the enzyme source can be used as the aqueous medium.

The dipeptide produced in the aqueous medium can be analyzed by a usual method using high performance liquid chromatography. The dipeptide can be collected from the aqueous medium by a conventional method using activated carbon or an ion exchange resin.

### 5-2. Method for Producing Dipeptide by Fermentation Process

An example of the method for producing a dipeptide of the present invention includes a method for producing a dipeptide by fermentation process in which the transformant described above in item 4, namely, a microorganism having ability to produce the protein described above in item (1) or (2), is cultured in a medium to produce and accumulate a dipeptide in a culture, and the dipeptide is collected from the culture. Here, the culture encompasses the cultured microorganism having the ability to produce the protein described above in item (1) or (2) and the medium, and contains the protein described above in item (1) or (2) having been expressed in the culture by the microorganism.

The transformant of the present invention to be used in the method for producing a dipeptide by fermentation process is preferably a transformant having ability to produce L-amino acid to be used as a substrate of the dipeptide.

A method for culturing the transformant described above in item 4 can be performed in accordance with a usual method employed in culturing a microorganism.

As the medium for culturing the transformant, either of a natural medium and a synthetic medium may be used as long as it contains a carbon source, a nitrogen source, inorganic salts or the like that the transformant can assimilate, and the transformant can be efficiently cultured therein.

The carbon source may be any source as long as the transformant can assimilate it, and sugars such as glucose, fructose, sucrose, syrup containing any of these, starch, and starch hydrolysate, organic acids such as acetic acid and propionic acid, and alcohols such as glycerol, ethanol, and propanol can be used.

As the nitrogen source, for example, ammonium salts of inorganic acids or organic acids such as ammonia, ammonium chloride, ammonium sulfate, ammonium acetate, and ammonium phosphate, other nitrogen-containing compounds, peptone, meat extract, yeast extract, corn steep liquor, casein hydrolysate, soybean cake, soybean cake hydrolysate, various fermented bacterial cells and digested materials thereof, and the like can be used.

As the inorganic salt, monopotassium phosphate, dipotassium phosphate, magnesium phosphate, magnesium sulfate, sodium chloride, ferrous sulfate, manganese sulfate, copper sulfate, calcium carbonate and the like can be used.

When the transformant used in the method for producing a dipeptide by fermentation process does not have ability to produce L-amino acid to be used as a substrate, the L-amino acid is added to the medium during the culturing.

Alternatively, when the transformant used in the method for producing a dipeptide by fermentation process does not have ability to produce L-amino acid to be used as a substrate, L-amino acid may be supplied to the transformant of the present invention by co-culturing, with the transformant of the present invention, a microorganism having ability to produce L-amino acid from sugar during the culturing instead of adding L-amino acid to the medium.

The culturing is preferably performed usually by shaking culture or under aerobic conditions such as aerated and agitated culture. A culturing temperature is usually 15 to 40°C, and a culturing time is usually 5 hours to 7 days. The pH of the culture fluid during the culturing is usually kept at 3.0 to 9.0. The pH is adjusted using an inorganic or organic acid, an alkaline solution, urea, calcium carbonate, ammonia, or the like.

Besides, during the culturing, an antibiotic such as ampicillin or tetracycline may be added to the medium if necessary. In culturing a microorganism obtained by transformation with an expression vector using an inducible promoter as a promoter, an inducer may be added to the medium if necessary. For example, in culturing a microorganism obtained by transformation with an expression vector using a lac promoter, isopropyl-β-D-thiogalactopyranoside (IPTG) or the like may be added, and in culturing a microorganism obtained by transformation with an expression vector using a trp promoter, indoleacrylic acid or the like may be added to the medium.

The dipeptide can be produced by performing the culturing described above, thereby producing and accumulating the dipeptide in the culture, and collecting the dipeptide from the culture.

The dipeptide thus obtained can be analyzed by a usual method using high performance liquid chromatography. The collection of dipeptide from the culture or the treated product of the culture can be performed by a usual method using activated carbon or an ion exchange resin. When the dipeptide is accumulated in a bacterial cell, the bacterial cell is crushed ultrasonically or the like, and the dipeptide can be collected, with activated carbon, an ion exchange resin or the like, from a supernatant obtained by removing the bacterial cell by centrifugation.

The dipeptide produced by the production method of the present invention is a dipeptide represented by the following formula (I):

R¹-R² (I)

wherein R¹ represents L-alanine, and R² represents an amino acid residue selected from L-glutamine, L-glutamic acid, glycine, L-valine, L-leucine, L-isoleucine, L-proline, L-phenylalanine, L-tryptophan, L-methionine, L-serine, L-threonine, L-cysteine, L-asparagine, L-tyrosine, L-lysine, L-arginine, L-histidine, and L-aspartic acid.

### Examples

Examples of the present invention will now be described, and it is noted that the present invention is not limited to these examples.

### [Analysis Example]

In each example, analysis and quantitative determination of L-alanyl-L-glutamine and L-alanyl-L-alanine were performed in accordance with the following procedures by HPLC (manufactured by Shimadzu Corporation).

### Analysis conditions:

Column: Inert Sustain 3 µm 3 mm x 150 mm (manufactured by GL Sciences Inc.)
Column temperature: 40°C
Mobile phase: 4.08 g/L of monopotassium phosphate, 6.07 g/L of sodium heptanesulfonate, and 125 mL/L of acetonitrile (pH 2.5, adjusted with phosphoric acid)
Flow rate: 0.4 mL/min
Detection wavelength: 210 nm

### [Example 1] Creation of Microorganism Expressing Mutant YwfE

### (1) Preparation of Bacillus subtilis 168 strain-derived L-amino Acid α Ligase (YwfE) Expression Vector

PCR was performed with a genomic DNA of *Bacillus subtilis* 168 strain prepared by a conventional method used as a template, and with DNAs consisting of nucleotide sequences set forth in SEQ ID NOS: 4 and 5 used as a primer set to obtain a DNA fragment of about 1.4 kbp including ywfE gene.

The ywfE fragment obtained as described above was linked to an expression vector pET-21a(+) (manufactured by Merck Millipore) with In-Fusion HD Cloning Kit (Takara Bio Inc.) to obtain an expression plasmid pET21a-ywfE.

### (2) Creation of Microorganism Expressing Mutant YwfE

With the plasmid pET21a-ywfE obtained as described above in item (1) used as a template, and with DNAs consisting of nucleotide sequences shown as "Primer Set" in any one of Tables 1 to 3 used as a primer set, 57 types in total of plasmids in each of which an amino acid residue at any one of positions 107, 108, and 110 in the amino acid sequence of YwfE was substituted with another amino acid were created with Prime STAR Mutagenesis Basal Kit (manufactured by Takara Bio Inc.).

When L-Asn at position 107 in the amino acid sequence of YwfE was to be substituted, the primer sets shown in Table 1 were used, when L-Asn at position 108 was to be substituted, the primer sets shown in Table 2 were used, and when L-Leu at position 110 was to be substituted, the primer sets shown in Table 3 were used.

**[Table 1]**

| Substituted Amino Acid Residue at position 107 | Primer Set | Mutation Point |
|---|---|---|
| L-Phe | SEQ ID NO: 6 and SEQ ID NO: 7 | N107F |
| L-Leu | SEQ ID NO: 8 and SEQ ID NO: 9 | N107L |
| L-Ile | SEQ ID NO: 10 and SEQ ID NO: 11 | N107I |
| L-Val | SEQ ID NO: 12 and SEQ ID NO: 13 | N107V |
| L-Ser | SEQ ID NO: 14 and SEQ ID NO: 15 | N107S |
| L-Pro | SEQ ID NO: 16 and SEQ ID NO: 17 | N107P |
| L-Thr | SEQ ID NO: 18 and SEQ ID NO: 19 | N107T |
| L-Ala | SEQ ID NO: 20 and SEQ ID NO: 21 | N107A |
| L-Tyr | SEQ ID NO: 22 and SEQ ID NO: 23 | N107Y |
| L-His | SEQ ID NO: 24 and SEQ ID NO: 25 | N107H |
| L-Gln | SEQ ID NO: 26 and SEQ ID NO: 27 | N107Q |
| L-Lys | SEQ ID NO: 28 and SEQ ID NO: 29 | N107K |
| L-Asp | SEQ ID NO: 30 and SEQ ID NO: 31 | N107D |
| L-Glu | SEQ ID NO: 32 and SEQ ID NO: 33 | N107E |
| L-Cys | SEQ ID NO: 34 and SEQ ID NO: 35 | N107C |
| L-Trp | SEQ ID NO: 36 and SEQ ID NO: 37 | N107W |
| L-Arg | SEQ ID NO: 35 and SEQ ID NO: 39 | N107R |
| Gly | SEQ ID NO: 40 and SEQ ID NO: 41 | N107G |
| L-Met | SEQ ID NO: 42 and SEQ ID NO: 43 | N107M |

**[Table 2]**

| Substituted Amino Acid Residue at position 108 | Primer Set | Mutation Site |
|---|---|---|
| L-Phe | SEQ ID NO: 44 and SEQ ID NO: 45 | N108F |
| L-Ile | SEQ ID NO: 46 and SEQ ID NO: 47 | N108I |
| L-Met | SEQ ID NO: 48 and SEQ ID NO: 49 | N108M |
| L-Val | SEQ ID NO: 50 and SEQ ID NO: 51 | N108V |
| L-Ser | SEQ ID NO: 52 and SEQ ID NO: 53 | N108S |
| L-Pro | SEQ ID NO: 54 and SEQ ID NO: 55 | N108P |
| L-Thr | SEQ ID NO: 56 and SEQ ID NO: 57 | N108T |
| L-Ala | SEQ ID NO: 58 and SEQ ID NO: 59 | N108A |
| L-Tyr | SEQ ID NO: 60 and SEQ ID NO: 61 | N108Y |
| L-His | SEQ ID NO: 62 and SEQ ID NO: 63 | N108H |
| L-Gln | SEQ ID NO: 64 and SEQ ID NO: 65 | N108Q |
| L-Lys | SEQ ID NO: 66 and SEQ ID NO: 67 | N108K |
| L-Asp | SEQ ID NO: 68 and SEQ ID NO: 69 | N108D |
| L-Glu | SEQ ID NO: 70 and SEQ ID NO: 71 | N108E |
| L-Cys | SEQ ID NO: 72 and SEQ ID NO: 73 | N108C |
| L-Trp | SEQ ID NO: 74 and SEQ ID NO: 75 | N108W |
| L-Arg | SEQ ID NO: 76 and SEQ ID NO: 77 | N108R |
| Gly | SEQ ID NO: 78 and SEQ ID NO: 79 | N108G |
| L-Leu | SEQ ID NO: 80 and SEQ ID NO: 81 | N108L |

**[Table 3]**

| Substituted Amino Acid Residue at position 110 | Primer Set | Mutation Site |
|---|---|---|
| L-Ile | SEQ ID NO: 82 and SEQ ID NO: 83 | L110I |
| L-Met | SEQ ID NO: 84 and SEQ ID NO: 85 | L110M |
| L-Val | SEQ ID NO: 86 and SEQ ID NO: 87 | L110V |
| L-Ser | SEQ ID NO: 88 and SEQ ID NO: 89 | L110S |
| L-Pro | SEQ ID NO: 90 and SEQ ID NO: 91 | L110P |
| L-Thr | SEQ ID NO: 92 and SEQ ID NO: 93 | L110T |
| L-Ala | SEQ ID NO: 94 and SEQ ID NO: 95 | L110A |
| L-Tyr | SEQ ID NO: 96 and SEQ ID NO: 97 | L110Y |
| L-His | SEQ ID NO: 98 and SEQ ID NO: 99 | L110H |
| L-Gln | SEQ ID NO: 100 and SEQ ID NO: 101 | L110Q |
| L-Asn | SEQ ID NO: 102 and SEQ ID NO: 103 | L110N |
| L-Lys | SEQ ID NO: 104 and SEQ ID NO: 105 | L110K |
| L-Asp | SEQ ID NO: 106 and SEQ ID NO: 107 | L110D |
| L-Glu | SEQ ID NO: 108 and SEQ ID NO: 109 | L110E |
| L-Cys | SEQ ID NO: 110 and SEQ ID NO: 111 | L110C |
| L-Trp | SEQ ID NO: 112 and SEQ ID NO: 113 | L110W |
| L-Arg | SEQ ID NO: 114 and SEQ ID NO: 115 | L110R |
| Gly | SEQ ID NO: 116 and SEQ ID NO: 117 | L110G |
| L-Phe | SEQ ID NO: 118 and SEQ ID NO: 119 | L110F |

The wild type YwfE expression plasmid pET21a-ywfE obtained as described above in item (1), and the 57 types in total of mutant YwfE expression plasmids having respective mutation sites described above were used to transform BL21(DE3)pLysS (manufactured by Merck Millipore) to obtain transformants having the respective plasmids.

### [Example 2] Production of Dipeptide using Mutant YwfE

Each of the 58 types in total of transformants obtained in Example 1 described above was cultured on an LB plate at 30°C for 24 hours, and the resultant was inoculated into a large test tube holding 5 mL of LB medium containing 100 mg/L of ampicillin to be shaking cultured at 30°C for 20 hours. Thereafter, 0.1 mL of the resultant culture fluid was inoculated into a flask holding 50 mL of LB medium containing 100 mg/L of ampicillin to be shaking cultured at 30°C. After bacterial cell OD600 reached a range of 0.4 to 0.6, isopropyl-β-D-thiogalactopyranoside (IPTG) was added thereto to a final concentration of 0.5 mM, followed by further shaking culture for 6 hours. After completing the culturing, the resultant culture fluid was centrifuged to obtain a bacterial cell.

From the thus obtained bacterial cell, a crude protein extract was obtained, and a wild type YwfE enzyme or each mutant YwfE enzyme was collected from the extract with His Buffer Kit (manufactured by GE Healthcare). The collected liquid was diluted with Buffer A [50 mM Tris-HCl (pH 8.0), 0.1 mM EDTA, and 10% glycerol], and the resultant was concentrated and desalted with Amicon Ultra-4 (manufactured by Merck Millipore) to obtain each purified YwfE enzyme.

Each of the purified wild type YwfE enzyme and purified mutant YwfE enzymes was used to produce L-alanyl-L-glutamine (hereinafter referred to as L-Ala-L-Gln) by the following method to compare the effects of the respective mutation sites.

### (1) Dipeptide Production using Mutant YwfE having L-Asn at position 107 Substituted

The purified wild type YwfE enzyme obtained as described above and the 19 types in total of purified mutant YwfE enzymes in which L-Asn at position 107 in the amino acid sequence of YwfE was substituted with each of amino acid residues shown in Table 1 were used to examine production of L-Ala-L-Gln by the enzymatic reaction.

0.1 mL of a reaction solution containing 22.5 µg of each of the purified enzymes, 100 mM Tris-HCl (pH 9.5), 30 mM magnesium sulfate, 30 mM ATP-disodium, 40 mM L-Ala, and 40 mM L-Gln was prepared, followed by a dipeptide synthesis reaction at 37°C for 1 hour. After the reaction, the thus produced dipeptides were analyzed by HPLC. The amounts (mM) of L-Ala-L-Gln and L-alanyl-L-alanine (hereinafter referred to as L-Ala-L-Ala) produced through the enzymatic reaction with each of the purified enzymes, and enzyme activities (L-Ala-L-Gln synthesizing activity ratio and L-Ala-L-Ala ratio) of each of the purified enzymes are shown in Table 4.

In Table 4, "L-Ala-L-Gln Synthesizing Activity Ratio" refers to a value obtained, from each mutant YwfE, by dividing an amount of L-Ala-L-Glan produced per unit time by a concentration (mM) of the purified enzyme in the reaction solution, assuming that a value of the wild type YwfE was 1.00. In Table 4, "L-Ala-L-Ala Ratio" refers to a value obtained, from each mutant YwfE, by dividing an amount (mM) of L-Ala-L-Ala by the amount (mM) of L-Ala-L-Gln produced, assuming that a value of the wild type YwfE was 1.0.

**[Table 4]**

| Mutation Site | L-Ala-L-Gln (mM) | L-Ala-L-Ala (mM) | L-Ala-L-Gln Synthesizing Activity Ratio | L-Ala-L-Ala Ratio |
|---|---|---|---|---|
| Wild type | 4.67 | 2.52 | 1.00 | 1.00 |
| N107F | 1.47 | 0.58 | 0.31 | 0.73 |
| N107L | 1.36 | 0.62 | 0.29 | 0.85 |
| N107I | 0.63 | 0.43 | 0.14 | 1.25 |
| N107V | 1.66 | 1.38 | 0.35 | 1.55 |
| N107S | 5.68 | 2.08 | 1.22 | 0.68 |
| N107P | 2.66 | 1.31 | 0.57 | 0.91 |
| N107T | 3.06 | 1.65 | 0.66 | 1.00 |
| N107A | 5.81 | 1.40 | 1.24 | 0.45 |
| N107Y | 0.90 | 0.60 | 0.19 | 1.23 |
| N107H | 8.90 | 3.12 | 1.91 | 0.65 |
| N107Q | 4.34 | 1.02 | 0.93 | 0.44 |
| N107K | 2.08 | 1.02 | 0.45 | 0.91 |
| N107D | 3.76 | 0.25 | 0.80 | 0.12 |
| N107E | 0.55 | 0.09 | 0.12 | 0.31 |
| N107C | 3.69 | 1.97 | 0.79 | 0.99 |
| N107W | 1.71 | 0.46 | 0.36 | 0.50 |
| N107R | 0.47 | 0.35 | 0.10 | 1.38 |
| N107G | 9.18 | 2.08 | 1.96 | 0.42 |
| N107M | 7.42 | 1.90 | 1.59 | 0.48 |

As a result, as shown in Table 4, the mutant YwfE in which L-Asn at position 107 of the amino acid sequence of YwfE was substituted with L-Ser, L-Ala, L-His, L-Gln, L-Asp, L-Glu, Gly, or L-Met showed a reduced production ratio of L-Ala-L-Ala as compared with that in the wild type YwfE. Among these mutants, mutant YwfE in which L-Asn at position 107 in the amino acid sequence of YwfE was substituted with L-Ser, L-Ala, L-His, Gly, or L-Met also showed an increased amount of L-Ala-L-Gln produced as compared with that in the wild type YwfE.

In this manner, use of mutant YwfE in which L-Asn at position 107 in the amino acid sequence of YwfE is substituted with L-Ser, L-Ala, L-His, Gly, or L-Met was found to reduce the production ratio of L-Ala-L-Ala as a by-product dipeptide and increase the amount of L-Ala-L-Gln produced as compared with those in the wild type YwfE.

### (2) Production of Dipeptide using Mutant YwfE having L-Asn at position 108 Substituted

The purified wild type YwfE enzyme obtained and the 19 types in total of purified mutant YwfE enzymes in which L-Asn at position 108 in the amino acid sequence of YwfE was substituted with each of the amino acid residues shown in Table 2 were used to examine production of L-Ala-L-Gln by the enzymatic reaction.

0.1 mL of a reaction solution containing 15 µg of each of the purified enzymes (whereas 13.6 µg for purified mutant YwfE enzyme substituted with L-Phe), 100 mM Tris-HCl (pH 9.5), 30 mM magnesium sulfate, 30 mM ATP-disodium, 40 mM L-Ala, and 40 mM L-Gln was prepared, followed by a dipeptide synthesis reaction at 37°C for 1 hour. After the reaction, the thus produced dipeptides were analyzed by HPLC. The amino acid residue at position 108 in YwfE used in each reaction, the amounts (mM) of L-Ala-L-Gln and L-Ala-L-Ala produced, and enzyme activities are shown in Table 5. In Table 5, "L-Ala-L-Gln Synthesizing Activity Ratio" and "L-Ala-L-Ala Ratio" are defined the same as described above in item (1).

**[Table 5]**

| Mutation Site | L-Ala-L-Gln (mM) | L-Ala-L-Ala (mM) | L-Ala-L-Gln Synthesizing Activity Ratio | L-Ala-L-Ala Ratio |
|---|---|---|---|---|
| Wild type | 3.11 | 1.76 | 1.00 | 1.00 |
| N108F | 5.35 | 1.82 | 1.72 | 0.60 |
| N108I | 1.05 | 0.87 | 0.34 | 1.47 |
| N108M | 3.78 | 1.23 | 1.22 | 0.57 |
| N108V | 2.43 | 1.01 | 0.78 | 0.74 |
| N108S | 4.74 | 1.19 | 1.52 | 0.45 |
| N108P | 2.36 | 0.46 | 0.84 | 0.35 |
| N108T | 5.48 | 1.95 | 1.76 | 0.63 |
| N108A | 5.50 | 0.93 | 1.77 | 0.30 |
| N108Y | 4.43 | 1.89 | 1.42 | 0.76 |
| N108H | 1.47 | 0.87 | 0.47 | 1.05 |
| N108Q | 1.45 | 0.92 | 0.47 | 1.11 |
| N108K | - | - | - | - |
| N108D | 0.21 | 0.12 | 0.07 | 1.01 |
| N108E | 0.15 | 0.08 | 0.05 | 0.96 |
| N108C | 3.07 | 1.05 | 0.99 | 0.61 |
| N108W | 3.97 | 2.33 | 1.28 | 1.04 |
| N108R | 0.53 | 0.24 | 0.17 | 0.80 |
| N108G | 3.08 | 0.88 | 0.99 | 0.51 |
| N108L | 3.31 | 1.06 | 1.06 | 0.57 |

As a result, the mutant YwfE in which L-Asn at position 108 in the amino acid sequence of YwfE was substituted with L-Phe, L-Met, L-Ser, L-Pro, L-Thr, L-Ala, L-Tyr, Gly, or L-Leu showed a reduced production ratio of L-Ala-L-Ala as compared with that in the wild type YwfE. Among these mutants, mutant YwfE in which L-Asn at position 108 in the amino acid sequence of YwfE was substituted with L-Phe, L-Met, L-Ser, L-Thr, L-Ala, L-Tyr, or L-Leu also showed an increased amount of L-Ala-L-Gln produced as compared with that in the wild type YwfE.

In this manner, use of mutant YwfE in which L-Asn at position 108 in the amino acid sequence of YwfE is substituted with L-Phe, L-Met, L-Ser, L-Thr, L-Ala, L-Tyr, or L-Leu was found to reduce the production ratio of L-Ala-L-Ala as a by-product dipeptide is reduced and increase the amount of L-Ala-L-Gln produced as compared with those in the wild type YwfE.

### (3) Production of Dipeptide using Mutant YwfE having L-Leu at position 110 Substituted

The purified wild type YwfE enzyme obtained as described above and the 19 types in total of purified mutant YwfE enzymes in which L-Leu at position 110 in the amino acid sequence of YwfE was substituted with each of amino acid residues shown in Table 3 were used to examine production of L-Ala-L-Gln by the enzymatic reaction.

0.1 mL of a reaction solution containing 22.5 µg of each of the purified enzymes, 100 mM Tris-HCl (pH 9.5), 30 mM magnesium sulfate, 30 mM ATP-disodium, 40 mM L-Ala, and 40 mM L-Gln was prepared, followed by a dipeptide synthesis reaction at 37°C for 1 hour. After the reaction, the thus produced dipeptides were analyzed by HPLC. The amino acid residue at position 110 of YwfE used in each reaction, the amounts (mM) of L-Ala-L-Gln and L-Ala-L-Ala produced, and enzyme activities are shown in Table 6. In Table 6, "L-Ala-L-Gln Synthesizing Activity Ratio" and "L-Ala-L-Ala Ratio" are defined the same as described above in item (1).

**[Table 6]**

| Mutation Site | L-Ala-L-Gln (mM) | L-Ala-L-Ala (mM) | L-Ala-L-Gln Synthesizing Activity Ratio | L-Ala-L-Ala Ratio |
|---|---|---|---|---|
| Wild type | 4.40 | 2.54 | 1.00 | 1.00 |
| L110I | 4.16 | 1.51 | 0.95 | 0.63 |
| L110M | 5.07 | 1.89 | 1.15 | 0.65 |
| L110V | 3.82 | 1.04 | 0.87 | 0.47 |
| L110S | 2.04 | 0.66 | 0.46 | 0.56 |
| L110P | 1.81 | 0.72 | 0.41 | 0.69 |
| L110T | 2.06 | 0.68 | 0.47 | 0.57 |
| L110A | 2.63 | 0.51 | 0.60 | 0.34 |
| L110Y | 2.65 | 1.02 | 0.60 | 0.67 |
| L110H | 2.56 | 0.64 | 0.58 | 0.43 |
| L110Q | 1.91 | 0.72 | 0.43 | 0.66 |
| L110N | 4.28 | 0.43 | 0.97 | 0.17 |
| L110K | 0.62 | 0.17 | 0.14 | 0.49 |
| L110D | 0.30 | 0.09 | 0.07 | 0.50 |
| L110E | 0.28 | 0.08 | 0.06 | 0.51 |
| L110C | 4.52 | 1.12 | 1.03 | 0.43 |
| L110W | 8.45 | 1.11 | 1.92 | 0.23 |
| L110R | 0.37 | 0.07 | 0.09 | 0.33 |
| L110G | 0.95 | 0.35 | 0.22 | 0.64 |
| L110F | 7.58 | 1.29 | 1.72 | 0.29 |

As a result, the mutant YwfE in which L-Leu at position 110 in the amino acid sequence of YwfE was substituted with L-Met, L-Val, L-Ala, L-Asn, L-Cys, L-Trp, or L-Phe showed a reduced production ratio of L-Ala-L-Ala as compared with that in the wild type YwfE. Among these mutants, mutant YwfE in which L-Leu at position 110 in the amino acid sequence of YwfE was substituted with L-Met, L-Cys, L-Trp, or L-Phe also showed an increased amount of L-Ala-L-Gln produced as compared with that in the wild type YwfE.

In this manner, use of mutant YwfE in which L-Leu at position 110 in the amino acid sequence of YwfE is substituted with L-Met, L-Cys, L-Trp, or L-Phe was found to reduce the production ratio of L-Ala-L-Ala as a by-product dipeptide and increase the amount of L-Ala-L-Gln produced as compared with those in the wild type YwfE.

### [Example 3] Production of Dipeptide by Fermentation Process using Microorganism having Mutant YwfE

### (1) Creation of Microorganism

With a plasmid pPE167 including the wild type YwfE described in Non Patent Literature 2 used as a template, and with DNAs consisting of nucleotide sequences shown as "Primer Set" in Table 7 used as a primer set, 10 types in total of mutant YwfE expression plasmids in each of which any one of positions 107, 108, and 110 in the amino acid sequence of YwfE was substituted with another amino acid were created with Prime STAR Mutagenesis Basal Kit (manufactured by Takara Bio Inc.).

The wild type YwfE expression plasmid pPE167 and the 10 types of mutant YwfE expression plasmids were used for transforming JKYPQ3 strain described in Non Patent Literature 2 to obtain transformants respectively having the respective plasmids.

**[Table 7]**

| Position | Substituted Amino Acid | Primer Set | Mutation Site |
|---|---|---|---|
| 107 | L-Ala | SEQ ID NO: 20 and SEQ ID NO: 21 | N107A |
| 107 | L-His | SEQ ID NO: 24 and SEQ ID NO: 25 | N107H |
| 107 | Gly | SEQ ID NO: 40 and SEQ ID NO: 41 | N107G |
| 108 | L-Ser | SEQ ID NO: 52 and SEQ ID NO: 53 | N108S |
| 108 | L-Thr | SEQ ID NO: 56 and SEQ ID NO: 57 | N108T |
| 108 | L-Ala | SEQ ID NO: 58 and SEQ ID NO: 59 | N108A |
| 108 | L-Tyr | SEQ ID NO: 60 and SEQ ID NO: 61 | N108Y |
| 110 | L-Met | SEQ ID NO: 84 and SEQ ID NO: 85 | L110M |
| 110 | L-Trp | SEQ ID NO: 112 and SEQ ID NO: 113 | L110W |
| 110 | L-Phe | SEQ ID NO: 118 and SEQ ID NO: 119 | L110F |

### (2) Production of Dipeptides

Each of the thus obtained transformants was cultured on an LB plate containing 100 mg/L of kanamycin at 30°C for 24 hours, the resultant was inoculated into a large test tube holding 5 mL of LB medium containing 10 g/L of glucose and 100 mg/L of kanamycin to be shaking cultured at 30°C for 20 hours. Thereafter, 0.1 mL of the resultant culture fluid was inoculated into a large test tube holding 5 mL of a production medium containing 100 mg/L of kanamycin [30 g/L of glucose, 1 g/L of magnesium sulfate heptahydrate, 5 g/L of casamino acid, 2 g/L of ammonium sulfate, 16 g/L of dipotassium hydrogen phosphate, 14 g/L of potassium dihydrogen phosphate, 1 g/L of trisodium citrate dihydrate, 0.4 g/L of L-proline, 10 mg/L of thiamine hydrochloride, 50 mg/L of ferrous sulfate heptahydrate, and 10 mg/L of manganese sulfate pentahydrate (wherein ingredients other than glucose and magnesium sulfate heptahydrate were adjusted to pH 7.2 with a sodium hydroxide aqueous solution to be autoclaved, an aqueous solution containing glucose and magnesium sulfate heptahydrate was separately prepared to be autoclaved, and the resultants were mixed with each other after cooling)], followed by shaking culture at 30°C for 48 hours.

After completing the culturing, the resultant culture fluid was centrifuged, and concentrations (g/L) of L-Ala-L-Gln and L-Ala-L-Ala in a supernatant were analyzed by HPLC. Results are shown in Table 8. In Table 8, "L-Ala-L-Ala/L-Ala-L-Gln" refers to a value (%) obtained by dividing an amount (g/L) of L-Ala-L-Ala produced by an amount (g/L) of L-Ala-L-Gln produced.

**[Table 8]**

| Mutation Site | L-Ala-L-Gln (g/L) | L-Ala-L-Ala (g/L) | L-Ala-L-Ala /L-Ala-L-Gln (%) |
|---|---|---|---|
| Wild type | 0.40 | 0.20 | 50 |
| N107A | 0.59 | 0.022 | 3.7 |
| N107H | 0.81 | 0.033 | 4.1 |
| N107G | 1.20 | 0.029 | 2.4 |
| N108S | 1.23 | 0.015 | 1.2 |
| N108T | 1.14 | 0.021 | 1.8 |
| N108A | 1.53 | 0.0049 | 0.32 |
| N108Y | 1.30 | 0.034 | 2.6 |
| L110M | 1.03 | 0.040 | 3.9 |
| L110W | 1.29 | 0.016 | 1.2 |
| L110F | 1.64 | 0.016 | 1.0 |

As a result, use of a microorganism having ability to express mutant YwfE obtained by substituting L-Asn at position 107 in YwfE with L-Ala, L-His, or Gly, substituting L-Asn at position 108 with L-Ser, L-Thr, L-Ala, or L-Try, and substituting L-Leu at position 110 with L-Met, L-Trp, or L-Phe was found to increase the amount of L-Ala-L-Gln produced, and reduce a ratio of by-product L-Ala-L-Ala to L-Ala-L-Gln, as compared with those in a microorganism expressing the wild type YwfE, and to improve substrate specificity of YwfE by these mutations.

### Industrial Applicability

According to the present invention, a protein having dipeptide synthesizing activity with improved substrate specificity, and a method in which the protein or a microorganism having ability to produce the protein is used to efficiently produce a target dipeptide while reducing a by-product dipeptide produced in addition to the target dipeptide are provided.

## Claims

1. A protein consisting of an amino acid sequence obtained by substituting one or more amino acid residues selected from the group consisting of amino acid residues at positions 107, 108, and 110 in an amino acid sequence set forth in SEQ ID NO: 2 with amino acid residues of the following [1] to [3], respectively, the protein having L-amino acid α ligase activity with improved substrate specificity as compared with an original protein having the amino acid sequence set forth in SEQ ID NO: 2:
[1] for the amino acid residue at position 107, an amino acid residue selected from the group consisting of L-serine, L-alanine, L-histidine, L-glutamine, L-aspartic acid, L-glutamic acid, glycine, and L-methionine;
[2] for the amino acid residue at position 108, an amino acid residue selected from the group consisting of L-phenylalanine, L-methionine, L-serine, L-proline, L-threonine, L-alanine, L-tyrosine, glycine, and L-leucine; and
[3] for the amino acid residue at position 110, an amino acid residue selected from the group consisting of L-methionine, L-valine, L-alanine, L-asparagine, L-cysteine, L-tryptophan, and L-phenylalanine.

2. A protein consisting of an amino acid sequence obtained by substituting one or more amino acid residues selected from the group consisting of amino acid residues corresponding to positions 107, 108, and 110 in an amino acid sequence of an original protein according to the following in [4] or [5] below with amino acid residues of the following [1'] to [3'], respectively, the protein having L-amino acid α ligase activity with improved substrate specificity as compared with the original protein:
[1'] for the amino acid residue at position 107, an amino acid residue selected from the group consisting of L-serine, L-alanine, L-histidine, L-glutamine, L-aspartic acid, L-glutamic acid, glycine, and L-methionine;
[2'] for the amino acid residue at position 108, an amino acid residue selected from the group consisting of L-phenylalanine, L-methionine, L-serine, L-proline, L-threonine, L-alanine, L-tyrosine, glycine, and L-leucine; and
[3'] for the amino acid residue at position 110, an amino acid residue selected from the group consisting of L-methionine, L-valine, L-alanine, L-asparagine, L-cysteine, L-tryptophan, and L-phenylalanine;
[4] a mutant protein consisting of an amino acid sequence obtained by deleting, substituting, inserting, and/or adding 1 to 20 amino acids in an amino acid sequence set forth in SEQ ID NO: 2, and having L-amino acid α ligase activity; and
[5] a homologous protein consisting of an amino acid sequence having 80% or more identity to the amino acid sequence set forth in SEQ ID NO: 2, and having L-amino acid α ligase activity.

3. A DNA encoding the protein according to claim 1 or 2.

4. A recombinant DNA comprising the DNA according to claim 3.

5. A transformant obtained by transforming a host cell with the recombinant DNA according to claim 4.

6. A method for producing a dipeptide, comprising, with the protein according to claim 1 or 2 used as an enzyme source, causing the enzyme source and two types of L-amino acids to be present in an aqueous medium, thereby producing and accumulating a dipeptide in the aqueous medium, and collecting the dipeptide from the aqueous medium.

7. A method for producing a dipeptide, comprising culturing, in a medium, a microorganism having ability to produce the protein according to claim 1 or 2, thereby producing and accumulating a dipeptide in a culture, and collecting the dipeptide from the culture.

8. The production method according to claim 6 or 7, wherein the dipeptide is represented by the following formula (I):
R¹-R² (I)
wherein R¹ represents L-alanine, and R² represents an amino acid residue selected from L-glutamine, L-glutamic acid, glycine, L-valine, L-leucine, L-isoleucine, L-proline, L-phenylalanine, L-tryptophan, L-methionine, L-serine, L-threonine, L-cysteine, L-asparagine, L-tyrosine, L-lysine, L-arginine, L-histidine, and L-aspartic acid.
